# EUROPEAN PATENT APPLICATION

(11) **EP 4 513 421 A1**
(43) Date of publication of application: **26.02.2025**
(21) Application number: 23791923.8
(22) Date of filing: 20.04.2023
(51) Int. Cl.: G06Q 50/10

(54) **INFORMATION PROCESSING DEVICE, INFORMATION PROCESSING METHOD, AND INFORMATION PROCESSING PROGRAM**

(30) Priority: 21.04.2022 JP 2022070171
(71) Applicant: ZOZO, Inc., Chiba-shi, Chiba, 263-0023 (JP)
(72) Inventor: IETA, Tsuyoshi, Chiba-shi, Chiba 263-0023 (JP); MINEMURA, Shunsuke, Chiba-shi, Chiba 263-0023 (JP); ANDO, Fuminori, Chiba-shi, Chiba 263-0023 (JP); YOSHIOKA, Shunya, Chiba-shi, Chiba 263-0023 (JP); HORI, Miyuki, Chiba-shi, Chiba 263-0023 (JP)
(74) Representative: Hoffmann Eitle
(86) International application number: PCT/JP2023/015815
(87) International publication number: WO 2023/204275

(57) **Abstract**

To propose a cosmetic product that is suitable for an individual skin problem of a user. An information processing apparatus (100) includes an estimation unit (132) and a providing unit (134). The estimation unit (132) estimates a degree of resolution of a skin problem of a user for each cosmetic product. The providing unit (134) provides information indicating a degree of resolution of a target cosmetic product based on the degree of resolution that is estimated by the estimation unit (132) for each cosmetic product.

## Description

### Field

The present invention relates to an information processing apparatus, an information processing method, and an information processing program.

### Background

In some cases, it may be difficult for a user to determine an appropriate cosmetic product as a means for resolving a skin problem. Conventionally, a technology for proposing a cosmetic product that is suitable for a skin or preference of the user is known.

### Citation List

### Patent Literature

Patent Literature 1: Japanese Laid-open Patent Publication No. 2001-001668

### Summary

### Technical Problem

However, in the conventional technology, there is room for further improvement in proposing a cosmetic product that is suitable for an individual skin problem of a user. For example, in the conventional technology, a degree of resolution of a skin problem is not taken into account, and therefore, it is impossible to make a proposal with a high degree of resolution of the skin problem.

The present application has been conceived in view of the foregoing situations, and an object of the present application is to provide a cosmetic product that is suitable for an individual skin problem of a user.

### Solution to Problem

An information processing apparatus according to the present disclosure includes: an estimation unit that estimates a degree of resolution of a skin problem of a user for each cosmetic product; and a providing unit that provides information indicating a degree of resolution of a target cosmetic product based on the degree of resolution that is estimated by the estimation unit for each cosmetic product.

### Advantageous Effects of Invention

According to one aspect of embodiments, it is possible to propose a cosmetic product that is suitable for an individual skin problem of a user.

### Brief Description of Drawings

FIG. 1 is a diagram illustrating a configuration example of an information processing system according to one embodiment.
FIG. 2 is a diagram illustrating an example of information processing according to one embodiment.
FIG. 3 is a diagram illustrating a configuration example of a terminal device according to one embodiment.
FIG. 4 is a diagram illustrating a configuration example of an information processing apparatus according to one embodiment.
FIG. 5 is a diagram illustrating an example of a skin information storage unit according to one embodiment.
FIG. 6 is a diagram illustrating an example of a feedback storage unit according to one embodiment.
FIG. 7 is a flowchart illustrating an example of the information processing according to one embodiment.
FIG. 8 is a hardware configuration diagram illustrating an example of a computer that implements functions of the information processing apparatus.

### Description of Embodiments

Modes (hereinafter, referred to as "embodiments") for carrying out an information processing apparatus, an information processing method, and an information processing program according to the present application will be described in detail below with reference to the drawings. The information processing apparatus, the information processing method, and the information processing program according to the present application are not limited by the embodiments below. In addition, in each of the embodiments below, the same components are denoted by the same reference symbols, and repeated explanation will be omitted.

### Embodiment

### 1. Configuration of information processing system

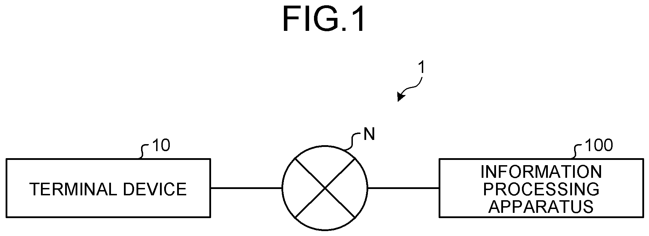

An information processing system 1 illustrated in FIG. 1 will be described below. As illustrated in FIG. 1, the information processing system 1 includes a terminal device 10 and an information processing apparatus 100. The terminal device 10 and the information processing apparatus 100 are communicably connected to each other in a wired or wireless manner via a predetermined communication network (network N). FIG. 1 is a diagram illustrating a configuration example of the information processing system 1 according to one embodiment.

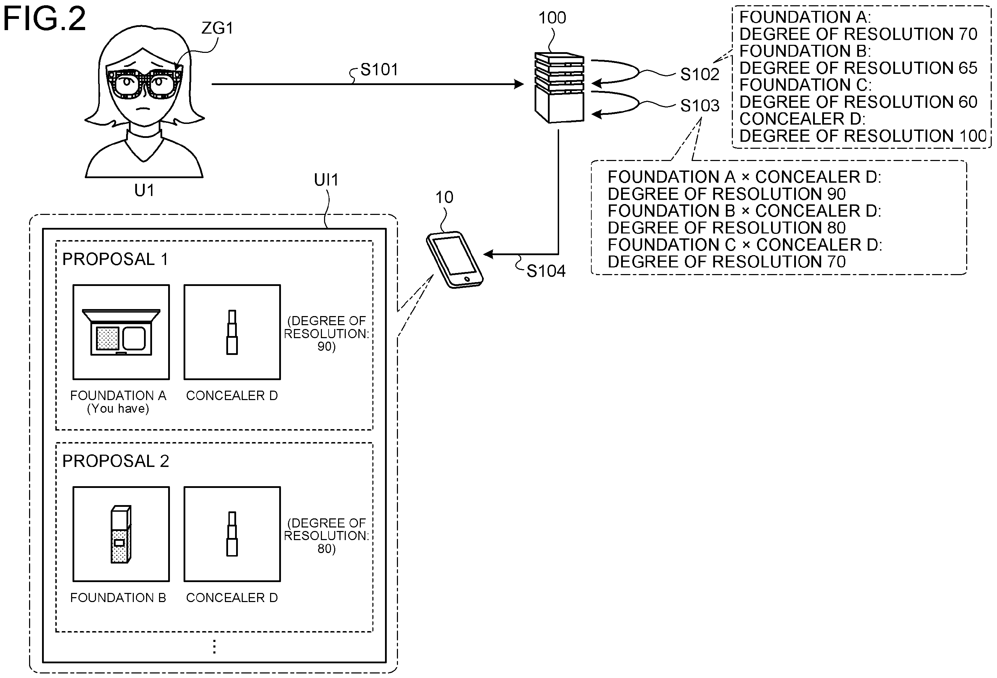

The terminal device 10 is an information processing apparatus that is used by a user who wants to resolve a skin problem. The terminal device 10 may be any device as long as it is possible to perform processes according to one embodiment. Further, the terminal device 10 may be a certain device, such as a smartphone, a tablet terminal, a notebook personal computer (PC), a desktop PC, a mobile phone, or a personal digital assistant (PDA). FIG. 2 illustrates a case in which the terminal device 10 is a smartphone.

The terminal device 10 is, for example, a smart device, such as a smartphone or a tablet, and is a mobile terminal device that is able to communicate with an arbitrary server apparatus via a wireless communication network, such as 3G to 5G (Generations) or LTE (Long Term Evolution). Further, the terminal device 10 may include a screen, such as a liquid crystal display, that has a function of a touch panel, and may receive various kinds of operation, such as tap operation, slide operation, or scroll operation, on displayed data, such as a content, by using a finger, a stylus, or the like from the user. In FIG. 2, the terminal device 10 is used by a user U1.

The information processing apparatus 100 is an information processing apparatus aimed at proposing a cosmetic product that is suitable for an individual skin problem of the user. The information processing apparatus 100 may be any apparatus as long as it is possible to perform processes according to one embodiment. For example, the information processing apparatus 100 may be implemented by a server apparatus, a cloud system, or the like that manages a predetermined service for providing a cosmetic product or a support.

### 2. Example of information processing

In some cases, it may be difficult for a user to determine an appropriate cosmetic product as a means for resolving a skin problem. For example, in some cases, it may be difficult to determine whether or not only a foundation or a makeup base is satisfactory, whether or not a cosmetic product with full coverage, such as a concealer, needs to be combined to cover a skin problem, or an appropriate combination of cosmetic products with full coverage, in order to cover a skin problem (for example, dark circles, stains, redness, pimples, or dullness) of the user.

The present invention has been conceived in view of the above-described circumstances, and an object of the present invention is to provide a cosmetic product that is suitable for an individual skin problem of the user.

FIG. 2 is a diagram illustrating an example of information processing performed by the information processing system 1 according to one embodiment. In the embodiment described below, a case will be described in which skin information, such as a skin color, is acquired from a ZOZOGLASS (registered trademark) ZG1, but a method of acquiring the skin information is not specifically limited. For example, the skin information may be input manually.

The information processing apparatus 100 acquires the skin information, such as a skin color, that is measured by ZG1 (Step S101). Meanwhile, the skin information is not limited to the information on the skin color, but includes, for example, information for identifying a skin problem or information for identifying a level of a symptom of a skin problem (for example, light, moderate, serious). The information processing apparatus 100 may acquire partial information of the skin information from ZG1, and may acquire remaining partial information based on, for example, manual input. For example, the information processing apparatus 100 may acquire the information on the skin color from ZG1, and acquire information for identifying a skin problem and information for identifying a level of a symptom of the skin problem based on, for example, manual input. Furthermore, the information processing apparatus 100 may estimate the skin problem of the user and the level of the symptom of the skin problem based on the acquired skin information, may estimate the skin problem of the user and the level of the symptom of the skin problem based on manual input, or may estimate the skin problem of the user and the level of the symptom of the skin problem by acquiring information (for example, a history of purchases of cosmetic products) that indicates a trend of the skin problem of the user.

The information processing apparatus 100 estimates a degree of resolution of the skin problem in a case where only a single cosmetic product is applied to cover the skin problem of the user (Step S102). For example, the information processing apparatus 100 estimates the degree of resolution of the skin problem in a case where only a foundation with the same color as the skin color of the user is applied. A method of estimating the degree of resolution of the skin problem will be described below. The information processing apparatus 100 estimates the degree of resolution of the skin problem based on, for example, how close a portion with the skin problem comes to a portion without the skin problem when the cosmetic product is applied. For example, the information processing apparatus 100 assumes that the portion without the skin problem is "100" and relatively estimates the degree of resolution of the skin problem when the cosmetic product is applied. For example, when the cosmetic product is applied, and if redness that is the skin problem is fully covered and the portion becomes equivalent to the portion without the skin problem, the information processing apparatus 100 estimates that the degree of resolution is "100". Further, for example, when the cosmetic product is applied and if coverage of the redness in the portion with the skin problem is 50% against the portion without the skin problem, the information processing apparatus 100 estimates that the degree of resolution is "50".

Meanwhile, in this case, the information processing apparatus 100 may absolutely estimate the degree of resolution of the skin problem in the portion with the skin problem, instead of based on the portion without the skin problem. For example, the information processing apparatus 100 may estimate that the degree of resolution is "50" when the cosmetic product is applied and the coverage of the redness that is the skin problem is 50% as compared to the case where the product is not applied.

Furthermore, as another estimation method, the information processing apparatus 100 may estimate the degree of resolution of the skin problem based on, for example, a level of satisfaction with a cosmetic product that the user has purchased in the past. For example, the information processing apparatus 100 may estimate the degree of resolution of the skin problem in a case where a cosmetic product that is similar to the cosmetic product that the user has purchased in the past is applied, in accordance with the level of satisfaction with the cosmetic product that the user has purchased in the past. For example, when the level of satisfaction with the cosmetic product that the user has purchased in the past is "100", the information processing apparatus 100 may estimate that the degree of resolution of the skin problem has been "100", and may estimate the degree of resolution of the skin problem with use of the similar cosmetic product is "100". Moreover, for example, the information processing apparatus 100 may estimate the degree of resolution of the skin problem in a case where a cosmetic product that is similar to a plurality of cosmetic products that the user has purchased in the past is applied, based on the levels of satisfaction with the plurality of cosmetic products that the user has purchased in the past. For example, when the level of satisfaction with a first cosmetic product that the user has purchased in the past is "100" and the level of satisfaction with a second cosmetic product that the user has purchased in the past is "50", the information processing apparatus 100 may estimate that the degrees of resolution of the skin problem are "100" and "50", and may estimate the degree of resolution of the skin problem with use of a similar cosmetic product that is similar to both of the first cosmetic product and the second cosmetic product is "75" that is an average.

In this case, the information processing apparatus 100 may acquire the skin information after application of the cosmetic product that the user has purchased in the past, store the degree of resolution of the skin problem and the level of satisfaction with the cosmetic product in an associated manner, and estimate the degree of resolution of the skin problem with use of the similar cosmetic product. For example, when the level of satisfaction with the cosmetic product that the user has purchased in the past is "100" and the degree of resolution of the skin problem based on the skin information after application of the cosmetic product is "80", the information processing apparatus 100 may estimate the degree of resolution of the skin problem with use of a similar cosmetic product that is similar to the cosmetic product for which the level of satisfaction is "100" is "80".

Explanation will be given below based on the assumption that the information processing apparatus 100 estimates the degree of resolution of the skin problem with use of a foundation A is "70", the degree of resolution of the skin problem with use of a foundation B is "65", the degree of resolution of the skin problem with use of a foundation C is "60", and the degree of resolution of the skin problem with use of a concealer D is "100".

The information processing apparatus 100 may estimate the degree of resolution of the skin problem in a case where a plurality of cosmetic products are applied to cover the skin problem of the user (Step S103). In the following, to estimate the degree of resolution of the skin problem in a case where a cosmetic product with light coverage and a cosmetic product with full coverage, such as a foundation and a concealer, are combined, it is assumed that at least one of the cosmetic products is a cosmetic product with light coverage, and at least another one of the cosmetic products is a cosmetic product with full coverage. A method of estimating the degree of resolution of the skin problem in a case where a plurality of cosmetic products are applied will be described below.

The information processing apparatus 100 may add, in advance, a weight to each category of a cosmetic product in accordance with the coverage, and may estimate the degree of resolution of the skin problem based on a combination. For example, it is assumed that a weight of a coverage of a category of a foundation is denoted by α, and a weight of a coverage of a category of a concealer is denoted by β. Meanwhile, it is assumed that α has a smaller value than β. Further, α and β may be weights that are determined by taking into account an order of application of the cosmetic products, such that the foundation is applied first and the concealer is applied later, for example. The information processing apparatus 100, when estimating the degree of resolution of the skin problem based on, for example, a combination of the foundation A and the concealer D, performs estimation based on "70" that is the degree of resolution of the skin problem with use of the foundation A, α that is the weight of the coverage of the category of the foundation, "100" that is the degree of resolution of the skin problem with use of the concealer D, and β that is the weight of the coverage of the category of the concealer.

Similarly, when estimating the degree of resolution of the skin problem based on a combination of the foundation B and the concealer D, the information processing apparatus 100 performs estimation based on, for example, "65" that is the degree of resolution of the skin problem with use of the foundation B, α that is the weight of the coverage of the category of the foundation, "100" that is the degree of resolution of the skin problem with use of the concealer D, and β that is the weight of the coverage of the category of the concealer. Furthermore, when estimating the degree of resolution of the skin problem based on a combination of the foundation C and the concealer D, the information processing apparatus 100 performs estimation based on, for example, "60" that is the degree of resolution of the skin problem with use of the foundation C, α that is the weight of the coverage of the category of the foundation, "100" that is the degree of resolution of the skin problem with use of the concealer D, and β that is the weight of the coverage of the category of the concealer.

Explanation will be given below based on the assumption that the information processing apparatus 100 estimates the degree of resolution of the skin problem with use of the foundation A and the concealer D is "90", the degree of resolution of the skin problem with use of the foundation B and the concealer D is "80", and the degree of resolution of the skin problem with use of the foundation C and the concealer D is "70".

Here, as a combination of a plurality of cosmetic products, if cosmetic products with largely different coverage are combined, makeup with a part of the cosmetic products may stand out. Further, even among foundations, coverage differ depending on types of the foundations (for example, cream, liquid, or powder). For example, in general, even among the foundations, coverage of a cream type is higher than a liquid type and a powder type, and coverage of the liquid type is higher than the powder type. Furthermore, even among concealers, coverage differ depending on types of the concealers (for example, pencil, stick, palette, liquid, or cream). For example, in general, even among concealers, coverage of a pencil type is higher than a stick type, a palette type, a liquid type, and a cream type, coverage of the stick type is higher than the palette type, the liquid type, and the cream time, coverage of the palette type is higher than the liquid type and the cream type, and coverage of the liquid type is higher than the cream type.

In this manner, even the cosmetic products in the same category have different coverage depending on types. Therefore, for example, when a powder type foundation and a pencil type concealer are combined, makeup with the pencil type may stand out in some cases. Therefore, the information processing apparatus 100 may limit cosmetic products to only a plurality of cosmetic products for which a difference in the coverage falls within a predetermined range, and may estimate the degree of resolution of the skin problem based on a combination. For example, when a powder type foundation is selected as one of cosmetic products to be combined, the information processing apparatus 100 may select a cream type concealer or a liquid type concealer for which a difference in the coverage with respect to the selected foundation falls within a predetermined range, and may estimate the degree of resolution of the skin problem based on a combination. In this case, the information processing apparatus 100 may select a concealer that is highly compatible with the selected foundation, a concealer that is highly compatible with the skin problem, or a concealer that is highly compatible with both of the selected foundation and the skin problem among concealers for which differences in the coverage with respect to the selected foundation falls within the predetermined range. Meanwhile, as for selection of one of the cosmetic products to be combined, for example, the one cosmetic product may be randomly selected from a purchase history of the user or may be selected based on preference information on the user, such as frequently purchased by the user, and not specifically limited.

Furthermore, the information processing apparatus 100 may add, in advance, a weight to each type of each category of a cosmetic product in accordance with the coverage, and may estimate the degree of resolution of the skin problem based on a combination. For example, it is assumed that a weight of a coverage of a powder type foundation is denoted by γ and a weight of a coverage of a liquid type concealer is denoted by ε. Meanwhile, magnitudes of values of γ and ε are not specifically limited. Moreover, γ and ε may be calculated based on a weight of each category of a cosmetic product and a weight of each type of the cosmetic product. Furthermore, γ and ε may be weights that are obtained by taking into account an order of application of the cosmetic products. When estimating the degree of resolution of the skin problem based on, for example, a combination of a powder type foundation and a liquid type concealer, the information processing apparatus 100 may perform estimation based on the degree of resolution of the skin problem with use of the powder type foundation, the weight γ of the coverage of the powder type foundation, the degree of resolution of the skin problem with use of the liquid type concealer, and the weight ε of the coverage of the liquid type concealer.

Moreover, the information processing apparatus 100 provides the user U1 with information indicating the degree of resolution of the skin problem based on the estimated degree of resolution of the skin problem for each cosmetic product or based on the estimated degree of resolution of the skin problem for each combination of a plurality of cosmetic products (Step S104). For example, the information processing apparatus 100 provides information for making a presentation in descending order of the degree of resolution of the skin problem. A screen UI1 is one example of a UI screen that is displayed on the terminal device 10. In this case, when the cosmetic products for which the degrees of resolution are presented include a cosmetic product that the user has purchased, it may be possible to display information indicating the purchased product, such as a "You have" tag. Furthermore, when the cosmetic products for which the degrees of resolution are presented include a cosmetic product that the user has purchased, it may be possible to preferentially display the purchased cosmetic product or a combination that includes the purchased cosmetic product. For example, it may be possible to first arrange the cosmetic products in descending order of the degree of resolution, and thereafter preferentially display the purchased cosmetic product or a combination that includes the purchased cosmetic product by selecting the purchased cosmetic product or the combination that includes the purchased cosmetic product as a presented cosmetic product. Moreover, for example, it may be possible to select the purchased cosmetic product or a combination that includes the purchased cosmetic product as a presented cosmetic product, and thereafter preferentially display the purchased cosmetic product or the combination that includes the purchased cosmetic product by arranging the products in descending order of the degree of resolution. Furthermore, it may be possible to display how the user looks when the presented cosmetic product is applied to the skin of the user, by using a technology for virtual fitting.

### First variation of process: presentation of cosmetic product with feedback taken into account

In the embodiment as described above, the case has been described in which the information processing apparatus 100 estimates a skin problem based on the skin information. In this case, the skin problem that is estimated by the information processing apparatus 100 and an actual skin problem of the user may be different from each other. The information processing apparatus 100 may perform a process of improving accuracy of estimation of a skin problem by asking the user whether the estimated skin problem is identical to the actual skin problem of the user. Further, the information processing apparatus 100 may perform a process of improving accuracy of estimation of a skin problem by finally obtaining a feedback on whether or not the skin problem is resolved after the user actually applies the presented cosmetic product. Furthermore, the information processing apparatus 100 may collect the feedbacks that are obtained as described above and perform a process of estimating the skin problem of the user based on a selection of a cosmetic product.

### Second variation of process: presentation of cosmetic product with continuous measurement result taken into account

In the embodiment as described above, the information processing apparatus 100 may perform a process of limiting an attribute (for example, a price) of a cosmetic product to be presented, by taking into account a continuous skin state. In this case, the continuous skin state may be estimated based on, for example, continuous measurement results of ZG1. For example, when a skin problem is not continuous (for example, when it is estimated that a cosmetic product will be applied only once), the information processing apparatus 100 may perform a process of selecting a presented cosmetic product from among cosmetic products that are available for purchase within a predetermined price. For example, the information processing apparatus 100 may perform a process of selecting, as the presented cosmetic product, a cosmetic product that is available for purchase within a predetermined price or a combination that includes the cosmetic product that is available for purchase within a predetermined price. Furthermore, for example, the information processing apparatus 100 may perform a process of selecting presented cosmetic products such that a total price of a plurality of cosmetic products falls within a predetermined price.

### Third variation of process: presentation of a cosmetic product with measurement result based on comparison with other people

In the embodiment as described above, the information processing apparatus 100 may perform a process of calculating an eigenvalue of a skin color of an individual user by comparison with other people based on, for example, measurement results of skin colors of a plurality of users collected from ZG1, determining compatibility with a method of estimation of a degree of resolution for resolving a skin problem, and making an appropriate presentation for the user. For example, when the skin color of the user is close to an average skin color of other people, the information processing apparatus 100 may perform a process of determining that the compatibility is high and making a presentation based on normal estimation. Furthermore, when, for example, the skin color of the user is brighter than the average skin color of other people, the information processing apparatus 100 may perform a process of selecting a presented cosmetic product while giving priority to a cosmetic product with light coverage as compared to normal estimation. In this manner, the information processing apparatus 100 may perform a process of preferentially presenting a cosmetic product that matches the eigenvalue of the skin color of the user or a combination that includes the etic product that matches the eigenvalue of the skin color of the user.

### Fourth variation of process: branching of process in accordance with degree of resolution of skin problem

In the embodiment as described above, the information processing apparatus 100 may determine whether or not to estimate the degree of resolution of the skin problem in a case where a plurality of cosmetic products are applied, in accordance with the degree of resolution of the skin problem in a case where only a single cosmetic product is applied. For example, when a single cosmetic product is present for which the degree of resolution of the skin problem exceeds a predetermined threshold in a case where only a single cosmetic product is applied, the information processing apparatus 100 may perform a process of making a presentation in descending order of the degree of resolution. For example, when the predetermined threshold is 62, the degrees of resolution of the skin problem with use of the foundations A and B among the foundations A to C exceed the predetermined threshold, and therefore, the information processing apparatus 100 may determine that estimation of the degree of resolution of the skin problem in a case where a plurality of cosmetic products are applied is not to be performed, and may perform a process of presenting the foundation A and the foundation B in this order. Furthermore, for example, when there is no cosmetic product for which the degree of resolution of the skin problem in a case where only a single cosmetic product is applied exceeds the predetermined threshold, the information processing apparatus 100 may perform a process of estimating the degree of resolution of the skin problem in a case where a plurality of cosmetic products are applied. For example, when the predetermined threshold is 75, the degree of resolution of the skin problem with use of each of the foundations A to C does not exceed the predetermined threshold, and therefore, the information processing apparatus 100 may perform a process of estimating the degree of resolution of the skin problem in a case where a plurality of cosmetic products are applied.

### 3. Configuration of terminal device

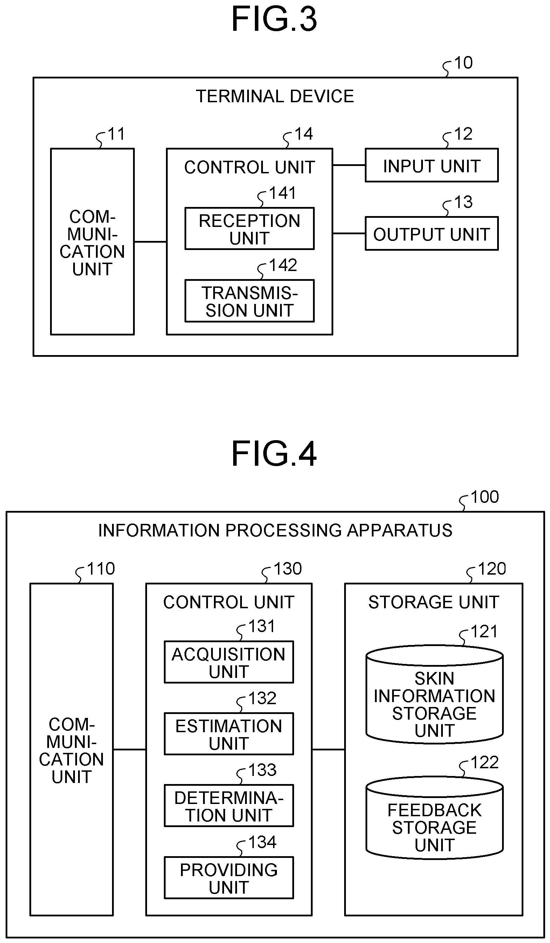

A configuration of the terminal device 10 according to one embodiment will be described below with reference to FIG. 3. FIG. 3 is a diagram illustrating a configuration example of the terminal device 10 according to one embodiment. As illustrated in FIG. 3, the terminal device 10 includes a communication unit 11, an input unit 12, an output unit 13, and a control unit 14.

### Communication unit 11

The communication unit 11 is implemented by, for example, a Network Interface Card (NIC) or the like. Further, the communication unit 11 is connected to the predetermined network N in a wired or wireless manner, and transmits and acquires information to and from the information processing apparatus 100 or the like via the predetermined network N.

### Input unit 12

The input unit 12 receives various kinds of operation from a user. In FIG. 2, various kinds of operation are received from the user U1. For example, the input unit 12 may receive various kinds of operation from the user via a display screen using a touch panel function. Further, the input unit 12 may receive various kinds of operation from a button that is arranged on the terminal device 10 or a keyboard or a mouse that is connected to the terminal device 10.

### Output unit 13

The output unit 13 is a display screen, such as a tablet terminal, that is implemented by, for example, a liquid crystal display or an organic Electro-Luminescence (EL) display, and is a display device for displaying various kinds of information. For example, the output unit 13 displays information that is transmitted from the information processing apparatus 100.

### Control unit 14

The control unit 14 is, for example, a controller, and is implemented by causing a Central Processing Unit (CPU), a Micro Processing Unit (MPU), or the like to execute various kinds of programs that are stored in a storage device in the terminal device 10 by using a Random Access Memory (RAM) as a work area. For example, the various kinds of programs include a program of an application that is installed in the terminal device 10. For example, the various kinds of programs include a program of an application that displays information indicating the degree of resolution of the skin problem based on the information that is transmitted from the information processing apparatus 100. Furthermore, the control unit 14 is implemented by, for example, an integrated circuit, such as an Application Specific Integrated Circuit (ASIC) or a Field Programmable Gate Array (FPGA).

As illustrated in FIG. 3, the control unit 14 includes a reception unit 141 and a transmission unit 142, and implements or executes information processing as described below.

### Reception unit 141

The reception unit 141 receives various kinds of information from a different information processing apparatus, such as the information processing apparatus 100. For example, the reception unit 141 receives information for displaying the information that indicates the degree of resolution of the skin problem. For example, the reception unit 141 receives information for making a presentation in descending order of the degree of resolution of the skin problem.

### Transmission unit 142

The transmission unit 142 transmits various kinds of information to a different information processing apparatus, such as the information processing apparatus 100. For example, the transmission unit 142 transmits information that is input in accordance with operation performed by the user, in order to request the information that indicates the degree of resolution of the skin problem. For example, the transmission unit 142 transmits information for identifying the skin problem or information for identifying a level of a symptom of the skin problem.

### 4. Configuration of information processing apparatus

A configuration of the information processing apparatus 100 according to one embodiment will be described below with reference to FIG. 4. FIG. 4 is a diagram illustrating a configuration example of the information processing apparatus 100 according to one embodiment. As illustrated in FIG. 4, the information processing apparatus 100 includes a communication unit 110, a storage unit 120, and a control unit 130. Meanwhile, the information processing apparatus 100 may include an input unit (for example, a keyboard, a mouse, or the like) that receives various kinds of operation from an administrator of the information processing apparatus 100, and a display unit (for example, a liquid crystal display or the like) for displaying various kinds of information.

### Communication unit 110

The communication unit 110 is implemented by, for example, a NIC or the like. Further, the communication unit 110 is connected to the network N in a wired or wireless manner, and transmits and acquires information to and from the terminal device 10 or the like via the network N.

### Storage unit 120

The storage unit 120 is implemented by, for example, a semiconductor memory device, such as a RAM or a flash memory, or a storage device, such as a hard disk or an optical disk. As illustrated in FIG. 4, the storage unit 120 includes a skin information storage unit 121 and a feedback storage unit 122.

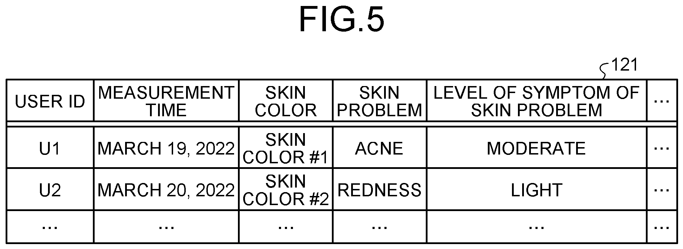

The skin information storage unit 121 stores therein skin information on the user. FIG. 5 illustrates an example of the skin information storage unit 121 according to one embodiment. As illustrated in FIG. 5, the skin information storage unit 121 includes items, such as a "user ID", a "measurement time", a "skin color", a "skin problem", and a "level of symptom of skin problem".

The "user ID" indicates identification information for identifying the user. The "measurement time" indicates a time at which the skin information is measured. The "skin color" indicates a skin color. In the example illustrated in FIG. 5, the example is illustrated in which conceptual information, such as a "skin color #1" or a "skin color #2" is stored in the "skin color"; however, in reality, a numerical value that indicates the skin color or the like is stored. Meanwhile, it may be possible to store a numerical value or the like that indicates a skin color of each part of the skin. The "skin problem" indicates a skin problem. Meanwhile, it may be possible to indicate a skin problem of each part of the skin. The "level of symptom of skin problem" indicates a level of a symptom of a skin problem. Meanwhile, it may be possible to indicate a level of a symptom of a skin problem of each part of the skin.

Specifically, FIG. 5 illustrates an example in which the skin information on the user who is identified by the user ID of "U1" is measured at a measurement time of "March 19, 2022", the skin color is the "skin color #1", the skin problem is "acne", and the level of the symptom of the skin problem is "moderate".

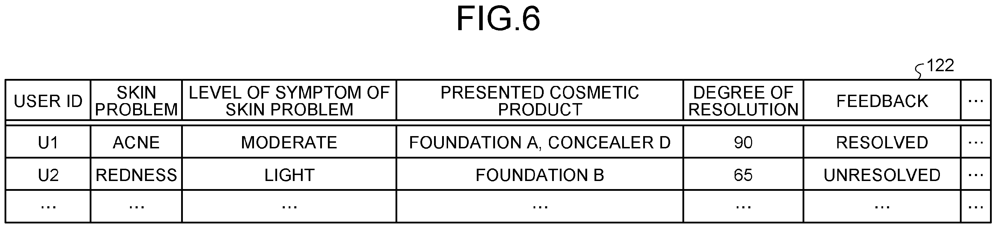

The feedback storage unit 122 stores therein feedback from the user. FIG. 6 illustrates an example of the feedback storage unit 122 according to one embodiment. As illustrated in FIG. 6, the feedback storage unit 122 includes items, such as a "user ID", a "skin problem", a "level of symptom of skin problem", a "presented cosmetic product", a "degree of resolution", and a "feedback".

The "user ID" indicates identification information for identifying the user. The "skin problem" indicates a skin problem. Meanwhile, it may be possible to indicate a skin problem of each part of the skin. The "level of symptom of skin problem" indicates a level of a symptom of the skin problem. Meanwhile, it may be possible to indicate the level of the symptom of the skin problem of each part of the skin. The "presented cosmetic product" indicates a presented cosmetic product. The "degree of resolution" indicates an estimated degree of resolution of the skin problem. The "feedback" indicates a feedback on whether or not the skin problem is resolved by actually applying the presented cosmetic product.

Specifically, FIG. 6 illustrates an example in which the skin problem of the user who is identified by the user ID of "U1" is "acne", the level of the symptom of the skin problem is "moderate", the presented cosmetic product is "foundation A, concealer D", the estimated degree of resolution of the skin problem is "90", and the feedback is "resolved".

### Control unit 130

The control unit 130 is, for example, a controller, and is implemented by causing a CPU, an MPU, or the like to execute various kinds of programs that are stored in a storage device in the information processing apparatus 100 by using a RAM as a work area. Further, the control unit 130 is a controller and implemented by, for example, an integrated circuit, such as an ASIC or an FPGA.

As illustrated in FIG. 4, the control unit 130 includes an acquisition unit 131, an estimation unit 132, a determination unit 133, and a providing unit 134, and implements or executes information processing as described below. Meanwhile, an internal configuration of the control unit 130 is not limited to the configuration as illustrated in FIG. 4, but it may be possible to adopt a different configuration as long as it is possible to perform the information processing (to be described later).

### Acquisition unit 131

The acquisition unit 131 acquires various kinds of information from an external information processing apparatus. The acquisition unit 131 acquires various kinds of information from a different information processing apparatus, such as the terminal device 10.

The acquisition unit 131 acquires various kinds of information from the storage unit 120. Further, the acquisition unit 131 stores various kinds of acquired information in the storage unit 120.

The acquisition unit 131 acquires the skin information, such as a skin color. For example, the acquisition unit 131 acquires the skin information based on a measurement result that is measured by ZG1 or the like. Further, for example, the acquisition unit 131 may acquire the skin information based on manual input. Furthermore, for example, the acquisition unit 131 may acquire information that indicates a trend of the skin problem of the user.

Moreover, the acquisition unit 131 may acquire a feedback on whether or not the skin problem is resolved. Meanwhile, the feedback may be a feedback based on evaluation indicating whether or not the problem is resolved (for example, resolved or unresolved), or may be a feedback based on evaluation indicating the degree of resolution (for example, resolved 90% of the problem or almost resolved).

### Estimation unit 132

The estimation unit 132 estimates the degree of resolution of the skin problem in a case where a single cosmetic product is used to cover the skin problem of the user. For example, the estimation unit 132 estimates the degree of resolution of the skin problem based on how close a portion with the skin problem comes to a portion without the skin problem when the cosmetic product is applied. Further, for example, the estimation unit 132 may absolutely estimate the degree of resolution of the skin problem in the portion with the skin problem, instead of based on the portion without the skin problem.
Furthermore, for example, the estimation unit 132 may estimate the degree of resolution of the skin problem based on the level of satisfaction with a cosmetic product that the user has purchased in the past. For example, the estimation unit 132 may estimate the degree of resolution of the skin problem when a cosmetic product that is similar to the cosmetic product that the user has purchased in the past is applied, in accordance with the level of satisfaction with the cosmetic product that the user has purchased in the past.

Moreover, the estimation unit 132 may estimate the degree of resolution of the skin problem in a case where a plurality of cosmetic products are applied to cover the skin problem of the user. For example, the estimation unit 132 may add, in advance, a weight to each category of a cosmetic product in accordance with the coverage, and may estimate the degree of resolution of the skin problem based on a combination. Furthermore, for example, the estimation unit 132 may add, in advance, a weight to each type of the category of the cosmetic product in accordance with the coverage, and may estimate the degree of resolution of the skin problem based on a combination. Moreover, for example, the estimation unit 132 may limit cosmetic products to only a plurality of cosmetic products for which a difference in the coverage falls within a predetermined range, and may estimate the degree of resolution of the skin problem based on a combination.

Furthermore, the estimation unit 132 may estimate the skin problem of the user or the level of the symptom of the skin problem based on the skin information that is acquired by the acquisition unit 131.

Moreover, the estimation unit 132 may estimate a continuous skin state based on, for example, a measurement result obtained by measurement using ZG1 or the like. Furthermore, the determination unit 133 (to be described later), when determining that the skin problem is not continuous based on an estimation result that is estimated by the estimation unit 132, may perform a process of limiting an attribute of a cosmetic product for which the degree of resolution is to be presented. For example, when cosmetic products for which the degrees of resolution are presented include a cosmetic product that exceeds a predetermined price, the determination unit 133 may perform a process of eliminating the cosmetic product or a combination that includes the cosmetic product from among the presented cosmetic products. Moreover, for example, the determination unit 133 may perform a process of preferentially presenting a cosmetic product that is available for purchase within a predetermined price or a combination that includes the cosmetic product that is available for purchase within the predetermined price.

### Determination unit 133

The determination unit 133 determines whether or not to estimate the degree of resolution of the skin problem in a case where a plurality of cosmetic products are applied, in accordance with the degree of resolution of the skin problem in a case where only a single cosmetic product is applied. For example, when determining that a single cosmetic product is present for which the degree of resolution of the skin problem exceeds a predetermined threshold in a case where only the single cosmetic product is applied, the determination unit 133 determines that a process for making a presentation in descending order of the degree of resolution is to be performed without estimating the degree of resolution of the skin problem in a case where a plurality of cosmetic products are applied. Furthermore, for example, when determining that a single cosmetic product is absent for which the degree of resolution of the skin problem exceeds a predetermined threshold in a case where only the single cosmetic product is applied, the determination unit 133 determines that the degree of resolution of the skin problem is to be estimated in a case where a plurality of cosmetic products are applied. Then, the estimation unit 132 estimates the degree of resolution of the skin problem in a case where a plurality of cosmetic products are applied in accordance with a determination result obtained by the determination unit 133.

Furthermore, the determination unit 133 may determine whether or not the presented cosmetic products include a cosmetic product that the user has purchased, based on, for example, a purchase history of the user. Moreover, when determining that the cosmetic product that the user has purchased is included, the determination unit 133 may perform a process of preferentially presenting the purchased cosmetic product or a combination that includes the purchased cosmetic product.

Moreover, the determination unit 133 may perform a process of calculating an eigenvalue of a skin color of an individual user by comparison with other people based on, for example, measurement results of skin colors of a plurality of users collected from ZG1 or the like, determining compatibility with a method of estimation of a degree of resolution for resolving a skin problem, and making an appropriate presentation for the user. For example, when the skin color of the user is close to an average skin color of other people, the determination unit 133 may perform a process of determining that the compatibility is high and making a presentation based on normal estimation. Furthermore, when, for example, the skin color of the user is brighter than the average skin color of other people, the determination unit 133 may perform a process of selecting a presented cosmetic product while giving priority to a cosmetic product with light coverage as compared to normal estimation. In this manner, the determination unit 133 may perform a process of preferentially presenting a cosmetic product that matches the eigenvalue of the skin color of the user or a combination that includes the cosmetic product that matches the eigenvalue of the skin color of the user.

### Providing unit 134

The providing unit 134 provides the user with information that indicates the degree of resolution of the skin problem based on the degree of resolution of the skin problem for each cosmetic product estimated by the estimation unit 132 or based on the estimated degree of resolution of the skin problem for each combination of a plurality of cosmetic products. For example, the providing unit 134 transmits information for making a presentation in descending order of the degree of resolution of the skin problem. Furthermore, when, for example, cosmetic products for which the degrees of resolution are presented include a cosmetic product that the user has purchased, the providing unit 134 may transmit information for displaying information indicating that the cosmetic product has already been purchased. Moreover, when, for example, cosmetic products for which the degrees of resolution are presented include a cosmetic product that the user has purchased, the providing unit 134 may transmit information for preferentially displaying the purchased cosmetic product or a combination that includes the purchased cosmetic product. Furthermore, for example, the providing unit 134 may transmit information for displaying how the user looks when the presented cosmetic product is applied to the skin of the user, by using a technology for virtual fitting.

### 5. Flow of information processing

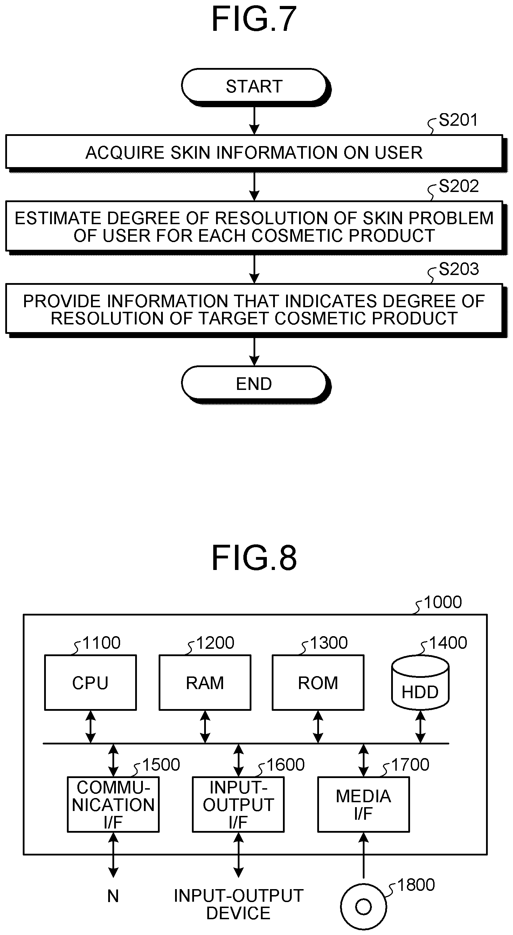

The flow of information processing performed by the information processing system 1 according to one embodiment will be described below with reference to FIG. 7. FIG. 7 is a flowchart illustrating the flow of the information processing performed by the information processing system 1 according to one embodiment.

As illustrated in FIG. 7, the information processing apparatus 100 acquires the skin information on the user (Step S201).

The information processing apparatus 100 estimates a degree of resolution of the skin problem of the user for each cosmetic product (Step S202).

The information processing apparatus 100 provides information that indicates the degree of resolution of a target cosmetic product (Step S203).

### 6. Modification

The information processing system 1 according to one embodiment as described above may be embodied in various different modes other than the embodiment as described above. Therefore, other embodiments of the information processing system 1 will be described below.

In the embodiment as described above, the case has been described in which the information processing apparatus 100 estimates the degree of resolution to cover a skin problem related to an appearance, such as dark circles, stains, redness, acne, or dullness, based on the skin information, such as a skin color. Here, the information processing apparatus 100 may estimate the degree of resolution to resolve a skin problem related to, for example, a texture, other than the appearance. In this manner, the information processing apparatus 100 may estimate the degree of resolution to resolve a skin problem as desired by the user, instead of the coverage. Furthermore, the information processing apparatus 100 may perform a process of presenting a cosmetic product with a high degree of resolution for resolving a skin problem as desired by the user, such that, for example, a texture is improved.

### 7. Effects

As described above, the information processing apparatus 100 according to one embodiment includes the estimation unit 132 and the providing unit 134. The estimation unit 132 estimates a degree of resolution of a skin problem of a user for each cosmetic product. The providing unit 134 provides information indicating a degree of resolution of a target cosmetic product based on the degree of resolution that is estimated by the estimation unit 132 for each cosmetic product.

With this configuration, the information processing apparatus 100 according to one embodiment is able to estimate the degree of resolution of the skin problem of the user for each cosmetic product and provide information based on the degree of resolution for each cosmetic product, so that it is possible to propose a cosmetic product that is suitable for an individual skin problem of the user.

Furthermore, the information processing apparatus 100 according to one embodiment further includes the determination unit 133 that determines whether or not a cosmetic present is present for which the degree of resolution that is estimated by the estimation unit 132 exceeds a predetermined threshold. Moreover, when the determination unit 133 determines that the cosmetic product is absent for which the degree of resolution exceeds the predetermined threshold, the estimation unit 132 estimates the degree of resolution for each combination of a plurality of cosmetic products.

With this configuration, when the degree of resolution is low only with use of a single cosmetic product, the information processing apparatus 100 according to one embodiment is able to provide information based on the degree of resolution for each combination of a plurality of cosmetic products, so that it is possible to propose a cosmetic product that is more suitable for an individual skin problem of the user.

Furthermore, the estimation unit 132 estimates the degree of resolution for each combination of a first cosmetic product that is selected based on one of a purchase history and preference information on the user and a second cosmetic product that is highly compatible with the first cosmetic product.

With this configuration, the information processing apparatus 100 according to one embodiment is able to estimate the degree of resolution while taking into account compatibility between cosmetic products, so that it is possible to make a proposal while taking into account the compatibility between the cosmetic products.

Moreover, the estimation unit 132 estimates the degree of resolution for each combination of the first cosmetic product and the second cosmetic product that is highly compatible with the skin problem of the user.

With this configuration, the information processing apparatus 100 according to one embodiment is able to estimate the degree of resolution while taking into account the compatibility with the skin problem of the user, so that it is possible to make a proposal while taking into account the compatibility with the skin problem of the user.

Furthermore, the providing unit 134 provides information for presenting cosmetic products in descending order of the degree of resolution that is estimated by the estimation unit 132.

With this configuration, the information processing apparatus 100 according to one embodiment is able to present cosmetic products that are suitable for an individual skin problem of the user in descending order of the degree of resolution, so that it is possible to promote improvement in usability.

Moreover, when the presented cosmetic products include a cosmetic product that the user has purchased based on whether or not the cosmetic product that the user has purchased is included, the providing unit 134 provides information for preferentially presenting one of the purchased cosmetic product and a combination that includes the purchased cosmetic product.

With this configuration, the information processing apparatus 100 according to one embodiment is able to preferentially present the cosmetic product that the user has purchased or a combination that includes the cosmetic product that the user has purchased, so that it is possible to promote improvement in usability.

Furthermore, when the skin problem of the user is not continuous based on whether or not the skin problem of the user is continuous, the providing unit 134 provides information for preferentially presenting a cosmetic product that is available for purchase within a predetermined price or a combination that includes the cosmetic product that is available for purchase within the predetermined price.

With this configuration, when the skin problem of the user is not continuous, the information processing apparatus 100 according to one embodiment is able to preferentially present the cosmetic product that is available for purchase within the predetermined price or the combination that includes the cosmetic product that is available for purchase within the predetermined price, so that it is possible to promote improvement in usability.

Moreover, when an eigenvalue of a skin color of the user is not average based on whether or not the eigenvalue of the skin color of the user calculated based on comparison with other people is average, the providing unit 134 provides information for preferentially presenting one of a cosmetic product that matches the eigenvalue of the skin color of the user and a combination that includes the cosmetic product that matches the eigenvalue of the skin color of the user.

With this configuration, when the eigenvalue of the skin color of the user is not average, the information processing apparatus 100 according to one embodiment is able to preferentially present the cosmetic product that matches the eigenvalue of the skin color of the user or the combination that includes the cosmetic product that matches the eigenvalue of the skin color of the user, so that it is possible to promote improvement in usability.

### 8. Hardware configuration

The terminal device 10 and the information processing apparatus 100 according to one embodiment as described above are implemented by, for example, a computer 1000 that is configured as illustrated in FIG. 8. FIG. 8 is a hardware configuration diagram illustrating an example of a computer that implements the functions of the terminal device 10 and the information processing apparatus 100. The computer 1000 includes a CPU 1100, a RAM 1200, a ROM 1300, an HDD 1400, a communication interface (I/F) 1500, an input-output interface (I/F) 1600, and a media interface (I/F) 1700.

The CPU 1100 operates based on a program that is stored in the ROM 1300 or the HDD 1400, and controls each of the units. The ROM 1300 stores therein a boot program that is executed by the CPU 1100 at the time of activation of the computer 1000, a program that is dependent on hardware of the computer 1000, or the like.

The HDD 1400 stores therein a program that is executed by the CPU 1100, data that is used by the program, or the like. The communication interface 1500 acquires data from a different apparatus via a predetermined communication network, sends the data to the CPU 1100, and transmits data that is generated by the CPU 1100 to a different apparatus via the predetermined communication network.

The CPU 1100 controls an output device, such as a display or a printer, and an input device, such as a keyboard or a mouse, via the input-output interface 1600. The CPU 1100 acquires data from the input device via the input-output interface 1600. Furthermore, the CPU 1100 outputs generated data to the output device via the input-output interface 1600.

The media interface 1700 reads a program or data that is stored in a recording medium 1800, and provides the program or the data to the CPU 1100 via the RAM 1200. The CPU 1100 loads the program from the recording medium 1800 onto the RAM 1200 via the media interface 1700, and executes the loaded program. The recording medium 1800 is, for example, an optical recording medium, such as a Digital Versatile Disc (DVD) or a Phase change rewritable Disk (PD), a magneto-optical recording device, such as a Magneto-Optical disk (MO), a tape medium, a magnetic recording medium, a semiconductor memory, or the like.

For example, when the computer 1000 functions as the terminal device 10 and the information processing apparatus 100 according to one embodiment, the CPU 1100 of the computer 1000 executes the program that is loaded on the RAM 1200 and implements the functions of the control units 14 and 130. The CPU 1100 of the computer 1000 reads the program from the recording medium 1800 and executes the program; however, as another example, it may be possible to acquire the program from a different device via the predetermined communication network.

### 9. Others

Of the processes described in the embodiments, all or part of a process described as being performed automatically may also be performed manually. Alternatively, all or part of a process described as being performed manually may also be performed automatically by known methods. In addition, the processing procedures, specific names, and information including various kinds of data and parameters illustrated in the above-described document and drawings may be arbitrarily changed unless otherwise specified. For example, various kinds of information illustrated in each of the drawings are not limited to the information as illustrated in the drawings.

Furthermore, the components of the apparatuses illustrated in the drawings are functionally conceptual and do not necessarily have to be physically configured in the manner illustrated in the drawings. In other words, specific forms of distribution and integration of the apparatuses are not limited to those illustrated in the drawings, and all or part of the apparatuses may be functionally or physically distributed or integrated in arbitrary units depending on various loads or use conditions.

Moreover, the embodiments as described above may be combined appropriately as long as processing contents do not conflict with one another.

While the embodiments of the present disclosure have been explained in detail above based on the drawings, the embodiments are described by way of example, and the present invention may be embodied in various other forms with various changes or modifications based on knowledge of a person skilled in the art, in addition to the embodiments described in this specification.

Furthermore, the "unit (section, module, unit)" described above may be replaced with a "means", a "circuit", or the like. For example, the acquisition unit may be replaced with an acquisition means or an acquisition circuit.

### Reference Signs List

- 1: information processing system

- 10: terminal device
- 11: communication unit
- 12: input unit
- 13: output unit
- 14: control unit
- 100: information processing apparatus
- 110: communication unit
- 120: storage unit
- 121: skin information storage unit
- 122: feedback storage unit
- 130: control unit
- 131: acquisition unit
- 132: estimation unit
- 133: determination unit
- 134: providing unit
- 141: reception unit
- 142: transmission unit
- N: network

## Claims

1. An information processing apparatus including:
an estimation unit that estimates a degree of resolution of a skin problem of a user for each cosmetic product; and
a providing unit that provides information indicating a degree of resolution of a target cosmetic product based on the degree of resolution that is estimated by the estimation unit for each cosmetic product.

2. The information processing apparatus according to claim 1, further including:
a determination unit that determines whether or not a cosmetic present is present for which the degree of resolution that is estimated by the estimation unit exceeds a predetermined threshold, wherein
when the determination unit determines that the cosmetic product is absent for which the degree of resolution exceeds the predetermined threshold, the estimation unit estimates the degree of resolution for each combination of a plurality of cosmetic products.

3. The information processing apparatus according to claim 2, wherein the estimation unit estimates the degree of resolution for each combination of a first cosmetic product and a second cosmetic product, the first cosmetic product being selected based on one of a purchase history and preference information on the user, the second cosmetic product being highly compatible with the first cosmetic product.

4. The information processing apparatus according to claim 3, wherein the estimation unit estimates the degree of resolution for each combination of the first cosmetic product and the second cosmetic product that is highly compatible with the skin problem of the user.

5. The information processing apparatus according to any one of claims 1 to 4, wherein the providing unit provides information for presenting cosmetic products in descending order of the degree of resolution that is estimated by the estimation unit.

6. The information processing apparatus according to claim 5, wherein when the presented cosmetic products include a cosmetic product that the user has purchased based on whether or not the cosmetic product that the user has purchased is included, the providing unit provides information for preferentially presenting one of the purchased cosmetic product and a combination that includes the purchased cosmetic product.

7. The information processing apparatus according to claim 5, wherein when the skin problem of the user is not continuous based on whether or not the skin problem of the user is continuous, the providing unit provides information for preferentially presenting one of a cosmetic product that is available for purchase within a predetermined price and a combination that includes the cosmetic product that is available for purchase within the predetermined price.

8. The information processing apparatus according to claim 5, wherein when an eigenvalue of a skin color of the user is not average based on whether or not the eigenvalue of the skin color of the user calculated based on comparison with other people is average, the providing unit provides information for preferentially presenting one of a cosmetic product that matches the eigenvalue of the skin color of the user and a combination that includes the cosmetic product that matches the eigenvalue of the skin color of the user.

9. An information processing method implemented by a computer, the information processing method including:
an estimation process of estimating a degree of resolution of a skin problem of a user for each cosmetic product; and
a providing process of providing information indicating a degree of resolution of a target cosmetic product based on the degree of resolution that is estimated at the estimating for each cosmetic product.

10. An information processing program that causes a computer to execute:
an estimation step of estimating a degree of resolution of a skin problem of a user for each cosmetic product; and
a providing step of providing information indicating a degree of resolution of a target cosmetic product based on the degree of resolution that is estimated at the estimating for each cosmetic product.
